(19) Europäisches Patentamt
European Patent Office
Office européen des brevets

(11) EP 1 029 897 A1

(12) **EUROPEAN PATENT APPLICATION**

(43) Date of publication:
**23.08.2000 Bulletin 2000/34**

(51) Int. Cl.[7]: **C08L 83/04**, C08G 77/32

(21) Application number: **00301224.2**

(22) Date of filing: **16.02.2000**

(84) Designated Contracting States:
**AT BE CH CY DE DK ES FI FR GB GR IE IT LI LU MC NL PT SE**
Designated Extension States:
**AL LT LV MK RO SI**

(30) Priority: **16.02.1999 JP 3674599**

(71) Applicant:
**SHIN-ETSU CHEMICAL CO., LTD.**
**Chiyoda-ku Tokyo (JP)**

(72) Inventors:
- **Nakanishi, Tersuo,**
**c/o Silicone-Elec. M. Res. Ctr.**
**Usui-gun, Gunma-ken (JP)**
- **Nezu, Sachiko,**
**c/o Silicone-Elec. Mat. Res. Ctr.**
**Usui-gun, Gunma-ken (JP)**
- **Sakuta, Koji,**
**c/o Silicone-Elec. Mat. Res. Ctr.**
**Usui-gun, Gunma-ken (JP)**
- **Masuyama, Yoshinobu,**
**c/o Gunma Complex**
**Annaka-shi, Gunma-Ken (JP)**

(74) Representative:
**Stuart, Ian Alexander et al**
**MEWBURN ELLIS**
**York House**
**23 Kingsway**
**London WC2B 6HP (GB)**

(54) **Organosilicon resin composition, method of making, and cosmetic composition containing the same**

(57) An organosilicon resin composition contains an organosilicon resin and a low molecular weight organopolysiloxane solvent which is a cyclic organopolysiloxane or a linear organopolysiloxane having a viscosity of up to 20 centistokes at 25°C. The composition has an aromatic hydrocarbon concentration of up to 10 ppm and is neutral in reaction when extracted with water so that it is useful in the area of cosmetics.

EP 1 029 897 A1

## Description

This invention relates to an organosilicon resin composition which is preferably free of aromatic hydrocarbon solvent and neutral in reaction, a method for preparing the same, and a cosmetic composition containing the same.

In the prior art, organosilicon or silicone resins are known as important intermediates for use in the preparation of silicone pressure-sensitive adhesives, silicone rubber compounds, and silicone parting agents. Recently, organosilicon resins are used in cosmetic compositions including foundations, lip colors, eye shadows, creams, emulsions and hair preparations for enhancing luster, water repellent, hair conditioning and other effects. The demand in the cosmetic application is increasing.

For example, organosilicon resins ace used in hair fixatives, which of ten take the form of hair cream or hair spray. In such hair preparations, various polymers (typically polyvinyl pyrrolidone) are compounded as the setting agent, and additives such as silicone, higher alcohols, liquid paraffin, ester oil and surfactants are blended in order to impart luster and smoothness. Despite their effect of imparting luster and smoothness, these additives have the serious drawback of significantly detracting from the hair conditioning capability. Silicone resins are added for an improvement in this regard.

The silicone resins used to this end include MQ resins comprising $R_3SiO_{1/2}$ units (M units wherein R is hydrogen or monovalent hydrocarbon group) and $SiO_2$ units (Q units); and MDQ or MTQ resins comprising $R_2SiO$ units (D units) or $RSiO_{3/2}$ units (T units) appropriately combined with M and Q units. For example, an MQ resin is prepared by starting with an organosilane from which M units are derived, such as $(CH_3)_3SiCl$, $(CH_3)_3SiOSi(CH_3)_3$, and $(CH_3)_3SiOH$ and a silicate from which Q units are derived, such as water glass, orthoalkyl silicates, and alkyl polysilicates, adding water and acid thereto along with an aromatic hydrocarbon solvent such as toluene and xylene, thereby effecting hydrolysis and condensation. After hydrolytic condensation, the acid is neutralized, and the alcohol is removed. Alternatively, the acid and alcohol are washed away with water. If neccssary, the aromatic hydrocarbon solvent is removed or exchanged with another solvent.

In the silicone resin thus produced, however, the aromatic hydrocarbon solvent can be left behind, which raises a serious problem in cosmetic applications, especially in the recent new applications intended to apply in proximity to mucous membrane such as lip colors and eye shadows. For this reason, the preparation of silicone resin intended for the cosmetic application requires careful removal of the solvent or exchange thereof with a cosmetically acceptable solvent. A long-term step of solvent removal or solvent exchange is required and a careful inspection is carried out at the end of preparation. It is desirable if a silicone resin could be prepared without using aromatic hydrocarbon solvent.

It is known from JP-A 5-345824 to prepare a silicone resin in an aliphatic hydrocarbon solvent rather than an aromatic hydrocarbon solvent. The silicone resin thus prepared, however, is liquid and its application is limited as compared with conventional silicone resins. Additionally, the aliphatic hydrocarbon solvent itself is yet inadequate as the solvent in the cosmetic application. especially iso-paraffin hydrocarbon solvents giving a smell.

Based on the discovery that the problem of JP-A 5-345824 is overcome by synthesizing a silicone resin in a silicone oil. JP-A 8-27272 provides an organosilicon resin composition substantially free of aromatic solvent.

In addition to the above considerations, silicone resins are required to be neutral in reaction when they are used in cosmetic compositions. The reaction of a material is one of inspection items considered important from the irritation-on-use standpoint as well as the stability of cosmetic compositions. However, the silicone resins synthesized by the above-described methods have a high viscosity and a large content of uncondensed silanol groups so that they cannot be effectively neutralized with an equivalent amount of aqueous alkali solution because of adsorption of acid catalyst. Thus most silicone resins produced are not neutral. Then the silicone resin systems will thicken with the lapse of time, smell when heated, lack stability, and irritate the skin.

It is thus desirable to provide an organosilicon resin composition which is free of an aromatic hydrocarbon solvent, neutral in reaction, stable and low irritant. It is also desirable to provide a method for preparing the organosilicon resin composition and a cosmetic composition containing the organosilicon resin composition.

An organosilicon resin is synthesized by a process (A) or subjecting a hydrolyzable silicon compound to hydrolysis and condonsation in the presence of an acid catalyst in a low molecular weight organopolysiloxane solvent or a process (B) of subjecting a hydrolyzable silicon compound to hydrolysis and condensation in the presence of an acid catalyst in a solventless system or in an aliphatic alcohol solvent. and adding thereto a low molecular weight organopolysiloxane solvent to effect solvent exchange. The hydrolytic condensation reaction in the presence of the acid catalyst is followed by removal of the acid. The acid is generally removod by neutralizing the reaction solution with an ammonium salt, carbonate salt, hydrogen carbonate salt or hydroxido of an alkali metal or alkaline earth metal or by washing the reaction solution with water. If the reaction solution is excessively alkaline, the alkali metal can adsorb on unreacted silanol groups, preventing the solution from becoming neutral even when an attempt to neutralize with hydrochloric acid or citric acid is made again. We have found that the organosilicon resin solution can be effectively controlled neutral by a process (1) for the neutralization of the acid after the synthesis of silicone resin wherein the rection solution

is previously and partially neutralized with a short amount of an aqueous solution of alkali metal hydroxide and further neutralized with a solid salt such as an alkali metal carbonate salt or a process (2) wherein the reaction solution is neutralized with a more than equivalent amount of an aqueous alkali solution so that the reaction solution becomes alkaline, the reaction solution is rendered weakly acidic again with a weak acid such as sodium citrate and then neutralized with an alkaline solid salt.

In a first aspect, the invention provides an organosilicon resin composition comprising an organosilicon resin of the following general formula (1) and a low molecular weight organopolysiloxane solvent which is a cyclic organopolysiloxane of the following general formula (2) or a linear organopolysiloxane having a viscosity of up to 20 centistokes at 25°C. The composition has an aromatic hydrocarbon concentration of up to 10 ppm and is neutral in reaction.

$$R^1_a(R^1O)_bSiO_{(4-a-b)/2} \qquad (1)$$

Herein $R^1$ is independently hydrogen or a substituted or unsubstituted monovalent hydrocarbon group of 1 to 10 carbon atoms, letter a is a positive number of from 0.1 to 3.0 and b is a positive number of from 0.01 to 0.5.

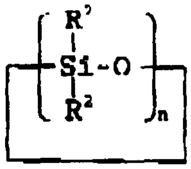

(2)

Herein $R^2$ is independently a substituted or unsubstituted monovalent hydrocarbon group, and n is a number of at least 4.

In a second aspect, the invention provides a method for preparing the organosilicon resin composition defined above. The method involves the step of subjecting a hydrolyzable silicon compound to hydrolysis and condensation in the presence of an acid catalyst in a low molecular weight organopolysiloxane solvent which is a cyclic organopolysiloxane of formula (2) or a linear organopolysiloxane having a viscosity of up to 20 centistokes at 25°C, thereby yielding a solution of the organosilicon resin of formula (1) in the low molecular weight organopolysiloxane solvent. Alternatively, the composition is prepared by subjecting a hydrolyzable silicon compound to hydrolysis and condensation in the presence of an acid catalyst in a solventless system or in an aliphatic alcohol solvent having 1 to 10 carbon atoms, and adding thereto a low molecular weight organopolysiloxane advent which is a cyclic organopolysiloxane of formula (2) or a linear organopolysiloxane having a viscosity of up to 20 centistokes at 25°C, to effect solvent exchange, thereby yielding a solution of the organosilicon resin of formula (1) in the low molecular weight organopolysiloxane solvent. In one embodiment of the method, the hydrolytic condensation is followed by the steps of neutralizing the acid catalyst with a short amount of an aqueous alkali solution, and neutralizing the residual weak acid with an alkaline solid salt. In another embodiment of the method, the hydrolytic condensation is followed by the steps of neutralizing the acid catalyst with an excess amount of an aqueous alkali solution, adding a weak acid to the solution so as to be acidic, and neutralizing the residual weak acid with an alkaline solid salt.

In a third aspect, there is provided a cosmetic composition comprising 0,01 to 50% by weight of the organosilicon resin composition of the first aspect or the organosilicon resin of formula (1) obtained by removing the solvent from the composition.

## DESCRIPTION OF THE PREFERRED EMBODIMENTS

The organosilicon resin composition of the invention contains as main components an organosilicon resin of the following general formula (1) and a low molecular weight organopolysiloxane serving as a solvent therefor which is selected from among a cyclic organopolysiloxane of the following general formula (2) and a linear organopolysiloxane having a viscosity of up to 20 centistokes at 25°C.

$$R^1_a(R^1O)_bSiO_{(4-a-b)/2} \qquad (1)$$

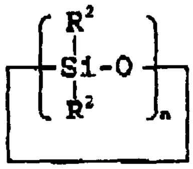

(2)

In formula (1) representing the organosilicon resin. $R^1$ which may be identical or different is hydrogen or a substituted or unsubstituted monovalent hydrocarbon group preferably of 1 to 10 carbon atoms. Illustrative examples include saturated aliphatic hydrocarbon groups such as methyl, ethyl, propyl, butyl and pentyl, saturated alicyclic hydrocarbon groups such as cyclopentyl and cyclohexyl, aromatic hydrocarbon groups such as phenyl and tolyl, and halogenated hydrocarbon groups such as trifluoropropyl, heptadecafluorodecyl, chloropropyl, and chlorophenyl. Preferred among others are saturated hydrocarbon groups of 1 to 5 carbon atoms, phenyl and trifluoropropyl.

In formula (1), letter a is a positive number Of from 0.1 to 3.0, preferably from 0.9 to 2.0, and b is a positive number of from 0.01 to 0.5, preferably from 0.01 to 0.2. Preferably these letters satisfy $0.01 < a+b < 3$, more preferably $0.1 \le a+b \le 2.4$ .

The organosilicon resin usually has a weight average molecular weight of about 500 to 100,000 and preferably about 1,000 to 50,000.

In formula (2) representing the cyclic organopolysiloxane, $R^2$ which may be identical or different is a substituted or unsubstituted monovalent hydrocarbon group, preferably having 1 to 10 carbon atoms, examples of which are the same as listed for $R^1$. Letter n is a number of at least 4, and preferably from 4 to 6. Preferred examples of the cyclic organopolysiloxane of formula (2) are octamethylcyclotetrasiloxane, decamethylcyclopentasiloxane. and dodecamethylcyclohexasiloxane.

Another low molecular weight organopolysiloxane is a linear organopolysiloxane having a viscosity of up to 20 centistokes at 25°C. Preferred examples or the linear organopolysiloxane are dimethylpolysiloxane and methylphenylpolysiloxane.

In the composition of the invention, the organosilicon resin of formula (1) should preferably be present in a concentration of 20 to 99% by weight, and more preferably 50 to 95% by weight.

In the composition, the concentration of aromatic hydrocarbons such as benzene and toluene should be up to 10 ppm and preferably up to 1 ppm.

Further, the composition should be neutral in reaction of extracting water. Specifically, 10 ml of ion-exchanged water is added to 10 g of the composition. The mixture is mildly boiled and allowed to cool down to room temperature. The extracting water should have pH in the range of 6 to 8. As used herein, the term "reaction" means the property that trio composition or resist exhibits when extracted with water.

The organosilicon resin of formula (1) in the organosilicon resin composition according to the invention can be synthesized by subjecting a hydrolyzable silicon compound to hydrolysis and condensation in the presence of an acid catalyst in a low molecular weight organopolysiloxane solvent or in a solventless system or in an aliphatic alcohol solvent of 1 to 10 carbon atoms.

The hydrolyzable silicon compounds used herein include hydrolyzable organosilanes such as $R^1_3SiOH$, $(R^1O)_2SiR^1_2$, and $(R^1O)_3SiR^1$ or partial hydrolytic condensates thereof; alkyl silicates represented by $(R^3O)_4Si$ such as $(CH_3O)_4Si$ and $(C_2H_5O)_4Si$, preferably $(C_2H_5O)_4Si$; organosiloxanes represented by $R^3_3SiOSiR^3_3$ such as $(CH_3)_3SiOSi(CH_3)_3$; and cyclotrisiloxanes represented by the following formula.

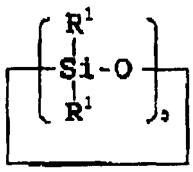

In the above formulae, $R^1$ is as defined above, and $R^3$ is a monovalent hydrocarbon group. Examples of the monovalent hydrocarbon group represented by $R^3$ are the same as listed for $R^1$.

When the hydrolyzable silicon compound undergoes hydrolysis and condensation in a low molecular weight organopolysiloxane advent or on aliphatic alcohol solvent, the amount of the solvent used is preferably 1 to 80% by weight of the entire system. The solvent and the hydrolyzable silicon compound are preferably used in a molar ratio of from 0.5:1 to 2:1.

Hydrolytic condensation is carried out under acidic conditions. As the catalyst, an acid is added to the system. The acids which can be used for hydrolysis include sulfuric acid, sulfurous acid, fuming sulfuric acid, p-toluenesulfonic acid, methanesulfonic acid, trifluoromethanesulfonic acid, hydrochloric acid, phosphoric acid, acetic acid, and citric acid. The acid may be used in a small amount, typically about 0.001 to 10% by weight of the entire system.

Useful processes of effecting hydrolytic condensation include two processes (A) and (B).

Process (A) involves charging a reactor with the hydrolyzable silicon compound and the low molecular weight organopolysiloxane solvent, adding the acid, and adding dropwise water while stirring the contents. Instead of first charging the reactor with the solvent, the solvent may be added after the completion of addition of water. During dropwise addition of water, the temperature is preferably maintained at 0 to 80°C , and especially 0 to 50°C. An appropriate amount of water added is in the range of 0.6:1 to 2:1 as expressed by the molar ratio of water to hydrolyzable alkoxy groups.

After the dropwise addition of water, the system is heated for about 2 to 8 hours at a temperature of 50 to 150°C, and preferably 80 to 120°C to effect hydrolytic condensation, which is followed by neutralized.

Process (B) involves subjecting at least one hydrolyzable silicon compound to hydrolytic condensation in a solventless system or in an aliphatic alcohol solvent of 1 to 10 carbon atoms, neutralizing the system, and then effecting solvent exchange with a low molecular weight organopolysiloxane solvent as defined above,

In process (B), hydrolytic condensation may be effected as in process (A). The solvent exchange may be effected in a conventional manner. One exemplary process involves the steps of charging a reactor with the hydrolyzable compound, adding the acid thereto, adding dropwise water while stirring the contents, effecting hydrolytic condensation in the absence of solvent or in an aliphatic alcohol solvent of 1 to 10 carbon atoms such as methanol or ethanol, then neutralizing the acid. Thereafter the low molecular weight organopolysiloxane solvent is added to the system preferably in an amount of 1 to 80%, more prefarably 10 to 50% by weight. Heat treatment is effected for 2 to 5 hours at 50 to 150°C, preferably 80 to 120°C, for thereby removing the formed alcohol, the alcohol solvent, and excess water, yielding a solution of the silicone resin in the low molecular weight organopolysiloxane solvent.

According to the invention, the hydrolysis is followed by acid removal or neutralization which can be performed in two ways. One type of neutralizing treatment according to the invention involves partially neutralizing the acid catalyst with a short amount of an aqueous alkali solution to render the system weakly acidic, and further neutralizing the residual weak acid with an alkaline solid salt. The amount of the aqueous alkali solution added is insufficient to neutralize the acidity of the remaining acid catalyst and desirably in the range of about 85 to 95% by weight based on the weight of the acid catalyst. The aqueous alkali solution is an aqueous solution of an alkali or alkaline earth metal, and preferably an aqueous solution of sodium hydroxide or potassium hydroxide. The alkaline solid salts which are weak bases include ammonium salts, carbonate salts and hydrogen carbonate salts of alkali metals, and ammonium salts, carbonate salts and hydrogen carbonate salts of alkaline earth metals. Of these salts, potassium carbonate is especially preferred. The salts are used in excess of an equivalent.

The other type of neutralizing treatment according to the invention involves the first step of neutralizing the acid catalyst with an excessive amount of an aqueous alkali solution, the second step of adding a weak acid so that the system may become acidic, and the third step of neutralizing the residual weak acid with an alkaline solid salt. The amount of an aqueous alkali solution used in the first step is more than the equivalent of the acid catalyst. After it is confirmed that the reaction solution is alkaline, the second step starts. The preferred weak acid is sodium citrate. The aqueous alkali solution and the alkaline solid salt used herein are the same as described above.

After the acid is neutralized in this way, the alcohol formed and excess water are removed by heating under atmospheric pressure or in vacuum. There is obtained a low molecular weight organopolysiloxane solution of the organosilicon resin of formula (1). It necessary, the solution is further heated in vacuum to remove the solvent, yielding an organosilicon resin composition according to the invention.

Since aromatic hydrocarbon solvents such as toluene and xylene are not used in the preparation process, the organosilicon resin composition thus obtained is free of such solvents. Specifically the concentration of aromatic hydrocarbon solvent is less than 10 ppm of the entire composition. The organosilicon resin has a high molecular weight and is less sticky and thus useful especially in cosmetic applications. The molecular weight distribution and shape of the organosilicon resin can be adjusted by changing the amounts of organosilane and alkyl silicate blended, the amount of catalyst added, reaction temperature, reaction time, the amount of solvent added, and addition procedure.

Since the specific type of neutralizing treatment is carried out, the resulting organosilicon resin composition is neutral in reaction (the property the resin exhibits when extracted with water). As a result, the composition is significantly less irritant to the skin, highly safe, and stable enough to prevent thickening with the lapse of time, Even when heated, the composition does not give a smell.

The organosilicon resin composition or the organosilicon resin of formula (1) obtained by distilling off the solvent therefrom can be effectively used as a cosmetic composition simply by blending appropriate pharmaceutically active ingredients and cosmetic ingredients therewith.

In the cosmetic composition, the amount of the organosilicon resin composition or organosilicon resin blended is 0.01 to 50% and preferably 0.05 to 20% by weight. Less than 0.01% by weight of the composition or resin is insufficient to impart a film-forming capability and silky feel. More than 50% by weight of the composition or resin detracts from applicability. If desired, a mixture of two or more organosilicon ream compositions may be used.

Various oily ingredients may be used in the cosmetic composition depending on its purpose. There may be used any of solid, semi-solid and liquid oily ingredients which are commonly used in conventional cosmetic compositions. Examples of natural animal and plant oils and fats and semi-synthetic oils and fats include avocado oil, linseed oil, almond oil, insect wax, perilla oil, olive oil, cacao butter, kapok oil, kaya oil, carnauba wax, liver oil, candelilla wax, beef tallow, beef foot oil, beef bone fat, hydrogenated beef tallow, apricot kernel oil, spermaceti, hydrogenated oil, wheat germ oil, sesame oil, rice germ oil, rice bran oil, sugar cane wax, sasanqua oil, safflower oil, shea butter, Chinese tang oil, cinnamon oil, jojoba wax, shellac wax, turtle oil, soybean oil, tea seed oil, tsubaki oil, evening primrose oil, corn oil, lard, rape seed oil, Japanese tung oil, rice bran wax, germ oil, horse fat, persio oil, palm oil, palm kernel oil, castor oil, hydrogenated castor oil, methyl castor oil fatty acid, sunflower seed oil, grape seed oil, bayberry wax, jojoba oil, macadamia nut oil, beeswax, mink oil, cotton seed oil, cotton wax, Japan wax, haze kernel oil, montan wax, coconut oil, hydrogenated coconut oil, tri-coconut oil fatty acid glyceride, mutton tallow, peanut oil, anhydrous lanolin, liquid lanolin, lanolin hydrogenated, lanolin alcohol, bard lanolin, lanolin acetate, lanolin fatty acid isopropyl, hexyl laurate, POE lanolin alcohol ether, POE lanolin alcohol acetate, polyethylene glycol lanolin fatty acid, POE hydrogenated lanolin alcohol ether, and egg yolk oil. Examples of hydrocarbon oils include ozokerite, squalane, squalane, ceresine, paraffin, paraffin wax, liquid paraffin, pristane, polyisobutyrene, microcrystalline wax, and vasoline. Exemplary higher fatty acids include laurie acid, myristic acid, palmitc acid, stearic acid, behenic acid, undecylenic acid, oleic acid, linolic acid, linoleic acid, arachidonic acid, eicosapentaenoic acid (EPA), docosahexaenoic acid (DHA), isostearic acid, and 12-hydroxystearic acid. Exemplary higher alcohols include lauryl alcohol, myristyl alcohol, stearyl alcohol, behenyl alcohol, hexadecyl alcohol, oleyl alcohol, isostearyl. alcohol, hexyldecanol, octyldodecanol, cetostearyl alcohol, 2-decyl totradecinol, cholesterol, phytosterol, POE cholesterol ether, glycerin monostearyl ether (batyl alcohol), and monooleyl glycerin ether (cerakyl alcohol). Exemplary ester oils include diisobutyl adipate, 2-hexyldecyl adipate, di-2-heptylundecyl adipate, N-alkylglyool monoisostearate, isocetyl isostearate, trimethylol propane triisostearate, ethylene glycol di-2-ethylhexanoate, cetyl 2-ethylhexanoate, trimethylol propane tri-2-ethylhexanoate, pentane erythritol tetra-2-ethylhexanoate, cetyl octanoate, octyldodecyl gum ester, oleyl oleate, octyldodecyl oleate, decyl oleate, dicapric acid neopentyl glycol, triethyl citrate, 2-ethylhexyl succinate, amyl acetate, ethyl acetate, butyl acetate, isocetyl stearate, butyl stearate, diisopropyl sebacate, di-2-ethylhexyl sebacate, cetyl lactate, myristyl lactate, isopropyl palmitate, 2-ethylhexyl palmitate, 2-hexyldecyl palmitate, 2-heptylundecyl palmitate, cholesteryl 12-hydroxylstearate, dipentaerythritol fatty acid ester, isopropyl myristate, 2-octyldodecyl myristate, 2-hexyldecyl myristate, myristyl myristate, hexyldecyl dimethyloctanoate, ethyl laurate, hexyl laurate, N-lauroyl-L-glutamic acid-2-octyldodeoyl ester, and malic acid diisostearyl. Exemplary glyceride oils include acctoglyceride, triisooctanoic acid glyceride, triisostearic acid glyceride, triisopalmitic acid glyceride, tri-2-ethylhexanoic acid glyceride, monostearic acid glyceride, di-2-heptylundecanoic acid glyceride, and trimyristic acid glyceride. Exemplary silicone oils include higher alkoxy-modified silicones and higher fatty acid-modified silicones such as dimethylpolysiloxane, methylphenylpolysiloxane, methylhydrogenpolysiloxane, octamethylcyclotetrasiloxane, decamethylcyclopentasiloxane, dodecamethylcyclohexasiloxane, tetramethyltetrahydrogencyclotetrasiloxane, and stearoxysilicone as well as silicone resins and silicone rubber. Exemplary fluorochemical oily ingredients include parfluoropolyether, perfluorodecalin, and perfluorooctane. These oily ingredients may be used alone or in admixture of two or more.

Various surfactants may be blended in the cosmetic composition of the invention depending on its purpose. The surfactants used herein include anionic, cationic, nonionic and ampholytic ones. The surfactant is not critical and selected from well-known surfactants which are used in conventional cosmetic compositions. Illustrative examples of anionic surfactants include fatty acid soaps such as sodium stearate and triethanol amine palmitate; alkyl ether carboxylic acids and salts thereof such as polyoxyethylene fatty alcohol ether carboxylate, carboxylic acid units such as condensates of amino acid with fatty acids; alkyl sulfonates, alkene sulfonates, sulfonic acid salts of fatty acid esters, sulfonic acid salts of fatty acid amides, alkylsulfonic acid salts and formalin condensates thereof, sulfuric acid ester salts such as alkyl sulfates, secondary higher alcohol sulfates, alkyl and allyl ether sulfates, fatty acid ester sulfates, fatty acid alkylol amide sulfates, and ether sulfates; alkyl phosphates, ether phosphates, alkyl allyl ether phosphates, amide phosphates, and N-acylamic acid. Illustrative examples of cationic surfactants include amine salts such as alkyl amine salts, polyamine and aminoalcohol fatty acid derivatives; alkyl quaternary ammonium salts, aromatic quaternary ammonium salts, pyridinium salt, and imidazolium salts. Illustrative examples of nonionic surfactants include sorbitol fatty acid esters, glycerin fatty acid esters, polyglycerin fatty acid esters, propylene glycol fatty acid esters, polyethylene glycol fatty acid esters, sucrose fatty acid esters, polyoxyethylene alkyl ethers, polyoxypropylene alkyl ethers, polyoxyethylene

alkyl phenyl ethers, polyoxyethylene fatty acid esters, polyoxyethylene sorbitol fatty acid esters, polyoxyethylene sorbitol fatty acid esters, polyoxyethylene glycerin fatty acid esters, polyoxyethylene propylene glycol fatty acid esters, polyoxyethylene castor oil, polyoxyethylene hardened castor oil, polyoxyethylene phytostanol ether, polyoxyethylene phytosterol ether, polyoxyethylene cholestanol ether, polyoxyethylene cholesteryl ether, polyoxyalkylene-modified organopolysiloxane, polyoxyalkylene/alkyl-co-modified organopolysiloxane, alkanol amides, saccharide ethers, and saccharide amides. Illustrative examples of ampholytic surfactants include betain, aminocarboxylic acid salts, and imidazoline derivatives.

Also powder is blended in the cosmetic composition. Any of well-known powders which are used in conventional cosmetic compositions may be used without restriction on its shape (inclusive of spherical, noodle and plate shapes), particle size (inclusive of fumes, microparticulates and pigment class size), and particle structure (inclusive of porous and nonporous). Exemplary inorganic powders include titanium oxide, sirconium oxide, zinc oxide, cerium oxide, magnesium oxide, barium sulfate, calcium sulfate, magnesium sulfate, calcium carbonate, magnesium carbonate, talc, mica, kaolin, sericite, muscovite, synthetic mica, phlogopite, lepidolite, biolite, lithia mica, silicic acid, silicic anhydride, aluminum silicate, magnesium silicate, aluminum magnesium silicate, calcium silicate, barium silicate, strontium silicate, metal salts of tungstenic acid, hydroxyapatite, vermiculite, haidilite, bentonite, montmorillonite, hectorite, zoolite, ceramics powder, calcium secondary phosphate, alumina, aluminum hydroxide, boron nitride, and silica. Exemplary organic powders include polyamide powder, polyester powder, polyethylene powder, polypropylene powder, polystyrene powder, polyurethane, benzoguanamine powder, polymethylbenzoguanamine powder. tetrafluoroothylene powder, polymethyl methacrylate powder, cellulose, silk powder, nylon powder, 12-nylon, 6-nylon, styrene-acrylic acid copolymers, divinyl benzene-styrene copolymers, vinyl resins, urea resins, phenolic resins, fluoro-resins, silicon resins, acrylic resins, melamine resins, epoxy resins, polycarbonate resins, microcrystalline fiber powder, rice starch, and lauroyl lysine. Suitable surfactant metal salt powders or metal soaps include zinc stearate, aluminum stearate, calcium stearate, magnesium stearate, zinc myristate, magnesium myristate, zinc cetyl phosphate, calcium cetyl phosphate, and zinc sodium cetyl phosphate. Suitable color pigments include inorganic red pigments much as iron oxide, iron hydroxide, and iron titanate; inorganic brown pigments such as $\gamma$-iron oxide; inorganic yellow pigments such as yellow iron oxide and ochre; inorganic black pigments such as black iron oxide and carbon black; inorganic purple pigments such as mango violet and cobalt violet; inorganic green pigments such as hydrated chromium oxide, chromium oxide, cobalt oxide, and cobalt titanate: inorganic blue pigments such as Iron blue and ultramarine, lake-form tar dyes, lake-form natural dyes, as well as synthetic resin powders obtained by compounding the foregoing powders. Suitable pearly pigments include titanium dioxide-coated mica, bismuth oxychloride, titanium dioxide - coated bismuth oxychloride, titanium dioxide-coated talc, fish scale foil, and titanium dioxide-coated color mica. Suitable metal powder pigments include aluminum powder, copper powder and stainless steel powder. Suitable tar dyes include Red No. 3. Red No. 104, Red No. 106, Red No. 201, Red No. 202, Red No. 204, Red No. 205, Red No. 220, Red No. 226, Red No. 227, Red No. 228, Red No. 230, Red No. 401, Red No. 505, Yellow No. 4, Yellow No. 5, Yellow No. 202, Yellow No. 203, Yellow No. 204, Yellow No. 401, Blue No. 1, Blue No. 2, Blue No. 201, Blue No. 404, Green No. 3, Green No. 201, Green No. 204, Green No. 205, Orange No. 201, Orange No. 203, Orange No. 204, Orange No. 206 and Orange No. 207 according to the rule of Ministry of Health and Welfare. Suitable natural dyes include carmiic acid, laccaic acid, carthamin, bradilin and crocin powders, which may be compounded or treated with ordinary oil ingredients. silicone oil, fluorine compounds or surfactants. Also acceptable are powders which are compounded or surface treated with oil ingredients, silicone or fluorine compounds. These powders may be used alone or in admixture of two or more.

In the cosmetic composition, other components which are used in conventional cosmetic compositions may be used insofar as the objects of the invention are not impaired. Such components include water, alcohols, water-soluble polymers, film-forming agents, oil-soluble gelling agents, organic modified clay minerals, resins, UV absorbers, humectants, preservatives, antiseptic agents, fragrances, salts, antioxidants, pH adjusting agents, chelating agents, refreshing agents, anti-inflammatory agents, skin-beautifying components, vitamins, amino acids, nucleic acid, hormones, and inclusion compounds.

Suitable alcohols include lower alcohols such as ethanol and isopropanol, and saccharide alcohols such as sorbitol and maltose. Suitable sterols include cholesterol, sitosterol, phytosterol and lanosterol.

Suitable water-soluble polymers include plant-origin polymers such as gum arabic, tragacanth gum, arabinogalactan, carob gum, gust gum, karaya gum, carrageenan, pectin, agar, quince seed, starch (rice, corn, potato and wheat), alge colloid, tranto gum, and locust bean gum; microorganism-origin polymers such as xanthan gum, dextran, succinoglucan, and pullulan; animal polymers such as collagen, casein, albumin, and gelatin; starch polymers such as carboxymethyl starch and methylhydroxypropyl starch; methyl cellulose, ethyl cellulose, methylhydroxypropyl cellulose, carboxymethyl cellulose, hydroxymethyl cellulose, hydroxypropyl cellulose, nitrocellulose, cellulose sodium sulfate, carboxymethyl cellulose sodium, microcrystalline cellulose, cellulosic polymers of cellulose powder, alginic acid polymers such as sodium alginate and propylene glycol alginate, vinyl polymers such as polyvinyl methyl ether and carboxyvinyl polymers, polyoxyethylene polymers, polyoxyethylene polyoxypropylene copolymers, acrylic polymers such as sodium polyacrylate, polyethyl acrylate and polyacrylamide, polyethylene imine, cationic polymers, and inorganic water-soluble

polymers such as bentonite, aluminum magnesium silicate, laponite, hectorite, and silicic anhydride. Also included are film-forming agents such as polyvinyl alcohol and polyvinyl pyrrolidone.

Suitable oil-soluble gelling agents include metal soaps such as aluminum stearate, magnesium stearate and zinc myristate, amino acid derivatives such as N-lauroyl-L-glutemic acid and α,γ-di-N-butylamine, dextrin fatty acid esters such as dextrin palmitate, dextrin stearate, and dextrin 2-ethylhexanoic acid palmitic acid ester, sucrose fatty acid esters such as sucrose palmitate and sucrose stearate, and benzylidene derivatives of sorbitol such as monobenzylidene sorbitol and dibenzylidene sorbitol. Also included are organic modified clay minerals such as dimethylbenzyldodecyl ammonium montmorillonite clay and dimethyldioctadecyl ammonium montmorillonite clay.

Suitable UV absorbers include benzoic acid UV absorbers much as p-aminobenzoic acid, anthranilic acid UV absorbers such as methyl anthranilate, salicylic acid UV absorbers such as methyl salicylate, cinnamic acid UV absorbers such as octyl p-methoxycinnamate, benzophenone UV absorbers such as 2,4-dihydroxybenzophenone, and urocanic acid UV absorbers such as ethyl urocanate.

Suitable humectants include sorbitol, xylytol, polyethylene glycol, hyaluronic acid, chondroitin sulfuric acid, and pyrrolidone carboxylic acid salt.

Suitable preservatives include p-oxybenzoic acid alkyl esters, benzoic acid, sodium benzoate, sorbic acid, potassium sorbate, and phenoxyethanol. Antiseptic agents include benzoic acid, salicylic acid, phenol, sorbic acid, p-oxybenzoic esters, p-chloro-m-cresol, hexachlorophene, benzalkonium chloride, chlorhexidine chloride, trichlorocarbanilide, photosensitizer, phenoxyethanol.

Suitable antioxidants include tocopherol, butylhydroxyanisol, dibutylhydroxytoluene, and γ-oryzanol. Suitable pH adjusting agents include lactic acid, citric acid, glycolic acid, succinic acid, tartaric acid, dl-malic acid, potassium carbonate, sodium hydrogen carbonate, and ammonium hydrogen carbonate. Suitable chelating agents include alanine, sodium salt of EDTA, sodium polyphosphate, sodium m-phosphate, and phosphoric acid. Suitable refreshing agents include L-menthol and camphor. Allantoin, glycyrrhetinic acid, tranexamic acid and azulene are also included in the anti-inflammatory agents.

Suitable skin beatifying agents include whitening agents such as placenta extract, albutin, glutathione, and saxifrage extract; cell activating agents and skin-protecting agents such as royal jelly, photosensitizer, cholesterol derivatives and bovine blood extract: blood stream promoters such as nonylic acid vanillylamide, benzyl nicotinate, β-butoxyethyl nicotinate, capsaicine, zingelon, cantharis tincture, ichthammol, caffeine, tannic acid, α-borneol, tocopherol nicotinate, inositol hexanicotinate, cyclandelate, cinnalysine, tolazoline, acetylcholine, verapamil, cepharanthine, and γ-oryzanol; skin astringents such as zinc oxide and tannic acid; and antiseborrheics such as such as sulfur and thianthol. Until vitamins include vitamin A compounds such as vitamin A oil, retinol, retinol acetate and retinol palmitate; vitamin $B_2$ compounds such as riboflavin, riboflavin lactate and flavin adenine nuclootide; vitamin $B_6$ compounds such as pyridoxine hydrochloride and pyridoxine dioctanoate; vitamin C compounds such as L-ascorbic acid, L-ascorbic acid dipalmitic acid ester. L-ascorbic acid-2-sulfuric acid sodium, and dl-α-tocopherol-L-ascorbic acid phosphoric acid diester dipotassium; pantothenic acid compounds such as calcium pantothenate, D-pantothenyl alcohol, pantothenyl ethyl ether and acetyl pantothenyl ethyl ether; vitamin D compounds such as ergocalciferol and cholecalciferol; nicotinic acid compounds such as nicotinic acid, benzyl nicotinate and nicotinic acid amide; vitamin E compounds such as dl-α-tocopherol, dl-α-tocopherol acetate, dl-α-tocopherol nicotinate and dl-α-tocopherol succinate; vitamin P, and biotin,

Suitable amino acids include alginine, aspartic acid, cystine, cysteine, methionine, cerine, leucine, and tryptophane. An exemplary nucleic acid is deoxyribonucleic acid. Suitable hormones include estradiol and ethenylestradiol.

As used herein, the cosmetic compositions include all preparations which are to be externally applied to the skin and must take into account their feel on use, typically external preparations and external drugs. Included are skin treating preparations such as lotions, emulsions, creams, face packs, massage creams, lip creams, hand creams, and cleansing creams; makeup preparations such as foundations, face powder, check colors, eye shadows, mascara, eye liners, eyebrow pencils, overcoats, anal lipsticks; nail makeup preparations such as nail enamels; and hair preparations such as rinses and treatments. These preparations may take any desired form including liquid, emulsion, solid, paste, and gel forms.

The organosilicon resin composition of the invention is substantially free of an aromatic hydrocarbon solvent which asates a safety problem, and neutral in reaction so that it is useful as a raw material in the area of cosmetics. A cosmetic composition containing the organosilicon resin composition is highly safe and less irritant because of a surprisingly low content of toluene as compared with prior art products.

## EXAMPLE

Examples of the invention are given below by way of illustration and not by way of limitation.

Example 1

A reactor was charged with 386 g of hexamethyldisiloxmne, 900 g of ethyl polysilicate having an $SiO_2$ content of 40%, and 580 g of octamethlcyclotetrasiloxane, to which 8 g of methanesulfonic acid was added. With stirring, 236 g of water was added dropwise to the reactor while controlling the temperature to 45 to 60°C. After the completion of dropwise addition, the solution was heated for 5 hours at 70 to 90°C for effecting hydrolysis. The reaction solution was neutralized with an excess amount of an aqueous sodium hydroxide solution and then rendered weakly acidic with sodium citrate. The acid was neutralized with calcium carbonate. The solution was heated at 120°C to distill off the ethanol formed and excess water. The solution was cooled, combined with 540 g of octamethylcyclotetrasiloxane, filtered, and heated at 100 to 120°C to effect vacuum distillation. The solution was cooled, filtered, and adjusted in volatile content, yielding 1,480 g of a 50% octamethylcyclotetrasiloxane solution of a silicone resin. As a result of molecular weight measurement by gel permeation chromatography (GPC), the silicone resin synthesized by this process was found to have a weight average molecular weight (MW) of 4,500.

Example 2

A reactor was charged with 386 g of hexamethyldisiloxane, 900 g of ethyl polysilicate having an $SiO_2$ content of 40%, and 386 g of isopropyl alcohol, to which 8 g of methanesulfonic acid was added. With stirring, 236 g of water was added dropwise to the reactor while controlling the temperature to 45 to 60°C. After the completion of dropwise addition, the solution was heated for 5 hours at 70 to 90°C for effecting hydrolysis. The reaction solution was neutralized with 4.4 g of an aqueous potassium hydroxide solution so as to be weakly acidic. The acid was neutralized with an excess amount of calcium carbonate. With 580 g of octamethylcyclotetrasiloxane added, the solution was heated at 120°C to distill off the ethanol formed and excess water. The solution was cooled, combined with 540 g of octamethylcyclotetrasiloxane, filtered, and heated at 100 to 120°C to effect vacuum distillation. The solution was cooled, filtered, and adjusted in volatile content, yielding 1,480 g of a 50% octamethylcyclotetrasiloxane solution of a silicone resin. As a result of molecular weight measurement by GPC, the silicons resin synthesized by this process was found to have a MW of 4,400.

Example 3

A reactor was changed with 386 g of hexamethyldisiloxane, 803 g of ethyl polysilicate having an $SiO_2$ content of 40%, 209 g of 3,3,3-trifluoropropyltriethoxysilane, and 386 g of isopropyl alcohol, to which 8 g of methanesulfonic acid was added. With stirring, 230 g of water was added dropwise to the reactor while controlling the temperature to 45 to 60°C. After the completion of dropwise addition, the solution was heated for 5 hours at 70 to 90°C for effecting hydrolysis. The reaction solution was neutralized with an excess amount of an aqueous potassium hydroxide solution and the excess alkali was neutralized with sodium citrate so that the reaction solution became weakly acidic. The acid was neutralized with calcium carbonate. With 423 g of octamethylcyclotetrasiloxane added, the solution was heated at 120°C to distill off the ethanol formed and excess water. The solution was cooled, combined with 320 g of decamethylcyclopentasiloxane, filtered, and heated at 100 to 120°C to effect vacuum distillation. The solution was cooled, filtered, and adjusted in volatile content, yielding 1,300 g of a 50% decamethylcyclopentasiloxane solution of a silicone resin. As a result of molecular weight measurement by GPC, the silicone resin synthesized by this process was found to have a MW of 5,200.

Comparative Example 1

A reactor was charged with 187 g of hexamethyldisiloxane, 440 g of ethyl polysilicate having an $SiO_2$ content of 40%, and 120 g of isopropyl alcohol, to which 6 g of methanesulfonic acid was added. With stirring, 118 g of water was added dropwise to the reactor while controlling the temperature to 45 to 60°C. After the completion of dropwise addition, the solution was heated for 5 hours at 70 to 90°C for effecting hydrolysis. After 130 g of toluene was added, the reaction solution was neutralized with an aqueous potassium hydroxide solution. The solution was heated at 120°C to distill off the ethanol formed and excess water. The solution was cooled, combined with 120 g of toluene, and filtered. After cooling, sodium carbonate was added to neutralize the excess of citric acid at 80 to 110°C. The solution was filtered and adjusted in volatile content, yielding 360 g of a 72% toluene solution of a silicone resin. As a result of molecular weight measurement by GPC, the silicone resin synthesized by this process was found to have a MW of 4,700.

A reactor was charged with 700 g of the silicone resin toluene solution (resin content 72%) and 520 g of octamethylcyclotetrasiloxane and heated to an internal temperature of 120°C to distill off the toluene in vacuum. The internal temperature was further raised to 130°C to distill off octamethylcyclotetrasiloxane. The solution was filtered and adjusted in volatile content, yielding 1,000 g of a 50% octamethylcyclotetrasiloxane solution of a silicone resin.

The silicone resin solutions obtained by the above procedures had physical properties as shown in Table 1. The solutions in Examples had a toluene content which was less than 1/10 of that of Comparative Example, indicating that the toluene content was nearly the detectable limit. The extracting water had pH in a substantially neutral range. It is thus evident that the silicone resins prepared by the inventive methods have chemical and physical properties adequate for use in cosmetic preparations.

Table 1

| | MW | Volatiles, 150°C/3hr | Extracting water pH* | Toluene content, ppm |
|---|---|---|---|---|
| EX1 | 4,500 | 49.2% | 7,5 | 1> |
| EX2 | 4,400 | 48.9% | 7.2 | 1> |
| EX3 | 5,200 | 47.2% | 7.1 | 1> |
| CE1 | 4,700 | 48.8% | 8.3 | 50 |

* The pH of extracting water was measured as previously described.

Example 4: Hair cream

| Ingredients | | Parts by weight |
|---|---|---|
| (a) | Octamethylcyclotetrasiloxane | 7 |
| (b) | Organosilicon resin composition of Example 1 | 6 |
| (c) | Liquid paraffin | 7 |
| (d) | Lanolin | 4 |
| (e) | Cetyl alcohol | 2 |
| (f) | Sorbitol monostearate | 2.5 |
| (g) | Polyoxyethylene (50) hydrogenated castor oil | 2 |
| (h) | Propylene glycol | 5 |
| (i) | Preservative | appropriate |
| (j) | Purified water | balance |

Formulation

Ingredients (a) to (g) were agitated and dissolved at 70 to 80°C to give a mixture (A). Ingredients (h) to (j) were dissolved to give a mixture (B). Mixture (A) was added to mixture (B) to form an emulsion which was a hair cream.

Example 5: Lip cream

| Ingredients | | Parts by weight |
|---|---|---|
| (a) | Squalane | 10 |
| (b) | Lanolin | 2 |
| (c) | Octamethylcyclotetrasiloxane | 34.7 |
| (d) | Isoparaffin | 10 |
| (e) | Organosilicon resin composition of Example 2 | 3 |
| (f) | Paraffin wax | 8 |
| (g) | Polyether-modified silicone* | 3 |
| (h) | Purified water | 3 |
| (i) | Glycerol | 25 |
| (j) | Sodium lactate | 0.3 |
| (k) | Sodium L-glutamate | 0.3 |
| (l) | Sodium hyaluronate | 0.1 |
| (m) | Sorbitol | 0.5 |
| (n) | Red pigment | 0.01 |
| (o) | Menthol | 0.1 |
| (p) | Fragrance | appropriate |

*Polyether-modified silicone is KF6017 commercially available from Shin-Etsu Chemical Co., Ltd.

Formulation

Oily ingredients were completely dissolved by heating at 80°C, whereupon the pigment was dispersed therein. An aqueous solution was added to this to form an emulsion, which was ready for use as lip cream after cooling.

Example 6: Oily eyeliner

| Ingredients | | Parts by weight |
|---|---|---|
| (a) | Carnauba wax | 5.5 |
| (b) | Bees wax | 9 |
| (c) | Microcrystalline wax | 9.5 |
| (d) | White vaseline | 1 |
| (e) | Liquid paraffin | 20 |
| (f) | Decamethylcyclopentasiloxane | 18 |
| (g) | Dimethylpolysiloxane | 32 |
| (h) | Organic modified bentonite | 0.5 |

(continued)

| Ingredients | | Parts by weight |
|---|---|---|
| (i) | Titanium oxide | 1.5 |
| (j) | Carbon black | 3 |
| (k) | Organosilicon resin composition of Example 3 | 5 |
| (l) | Preservative | appropriate |

Formulation

Oily ingredients were completely dissolved by heating at 80°C, whereupon the powder was dispersed therein. The aspersion was cooled to give the end oily eyeliner.

Japanese Patent Application No. 11-036745 is incorporated herein by reference.

Although some preferred embodiments have been described, many modifications and variations may be made thereto in light of the above teachings. It is therefore to be understood that the invention may be practiced otherwise than as specifically described without departing from the scope of the appended claims.

## Claims

**1.** An organosilicon roam composition comprising

an organosilicon resin of the following general formula (1):

$$R^1_a(R^1O)_bSiO_{(4-a-b)/2} \quad (1)$$

wherein $R^1$ is independently hydrogen or a substituted or unsubstituted monovalent hydrocarbon group of 1 to 10 carbon atoms, letter a is a positive number of from 0.1 to 3.0 and b is a positive number of from 0.01 to 0.5, and
an organopolysiloxane solvent
selected from a cyclic
organopolysiloxane of the following general formula (2):

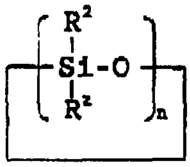

(2)

wherein $R^2$ is independently a substituted or unsubstituted monovalent hydrocarbon group, and n is a number of at least 4, and a linear organopolysiloxane having a viscosity of up to 20 centistokes at 25°C,
said composition having an aromatic hydrocarbon concentration of up to 10 ppm and being neutral in reaction.

**2.** A method for preparing an organosilicon resin composition as set forth in claim 1, comprising the step of subjecting a hydrolyzable silicon compound to hydrolysis and condensation in the presence of an acid catalyst in an

organopolysiloxane solvent selected from
a cyclic organopolysiloxane of the following general formula (2):

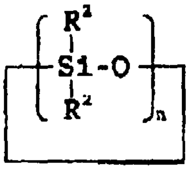

(2)

wherein $R^2$ is independently a substituted or unsubstituted monovalent hydrocarbon group, and n is a number of at least 4, and a linear organopolysiloxane having a viscosity of up to 20 centistokes at 25°C, thereby yielding a solution in the organopolysiloxane solvent of an organosilicon resin of the following general formula (1):

$$R^1_a(R^1O)_bSiO_{(4-a-b)/2} \quad (1)$$

wherein $R^1$ is independently hydrogen or a substituted or unsubstituted monovalent hydrocarbon group of 1 to 10 carbon atoms, letter a is a positive number of from 0.1 to 3.0 and b is a positive number of from 0.01 to 0.5, and wherein
the hydrolytic condensation is followed by the steps of neutralizing the acid catalyst with a short amount of an aqueous alkali, solution, and neutralizing the residual weak acid with an alkaline solid salt.

**3.** A method for preparing an organosilicon resin composition as set forth in claim 1, comprising the steps of subjecting a hydrolyzable silicon compound to hydrolysis and condensation in the presence of an acid catalyst in a solventless system or in an aliphatic alcohol solvent having 1 to 10 carbon atoms, and adding thereto an

organopolysiloxane solvent selected from
a cyclic organopolysiloxane of the following general formula (2):

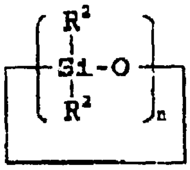

(2)

wherein $R^2$ is independently a substituted or unsubstituted monovalent hydrocarbon group, and n is a number of at least 4, and a linear organopolysiloxane having a viscosity of up to 20 centistokes at 25°C, to effect solvent exchange, thereby yielding a solution in the organopolysiloxane solvent of an organosilicon resin of the following general formula (1):

$$R^1_a(R^1O)_bSiO_{(4-a-b)/2} \quad (1)$$

wherein $R^1$ is independently hydrogen or a substituted or unsubstituted monovalent hydrocarbon group of 1 to 10 carbon atoms, letter a is a positive number of from 0.1 to 3.0 and b is a positive number of from 0.01 to 0.5, and wherein
the hydrolytic condensation is followed by the steps of neutralizing the acid catalyst with a short amount of an aqueous alkali solution, and neutralizing the residual weak acid with an alkaline solid salt.

**4.** A method for preparing an organosilicon resin composition as set forth in claim 1, comprising the step of subjecting a hydrolyzable silicon compound to hydrolysis and condensation in the presence of an acid catalyst in an

organopolysiloxane solvent selected from
a cyclic organopolysiloxane of the following general formula (2),

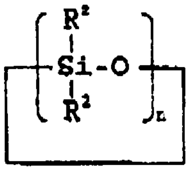

(2)

wherein $R^2$ is independently a substituted or unsubstituted monovalent hydrocarbon group, and n is a number of at least 4, and a linear organopolysiloxane having a viscosity of up to 20 centistokes at 25°C, thereby yielding a solution in the organopolysiloxane solvent of an organosilicon resin of the following general formula (1):

$$R^1_a(R^1O)_bSiO_{(4-a-b)/2} \quad (1)$$

wherein $R^1$ is independently hydrogen or a substituted or unsubstituted monovalent hydrocarbon group of 1 to 10 carbon atoms, letter a is a positive number of from 0.1 to 3.0 and b is a positive number of from 0.01 to 0.5, and wherein
the hydrolytic condensation is followed by the steps of neutralizing the acid catalyst with an excess amount of an aqueous alkali solution, adding a weak acid to the solution so as to be acidic, and neutralizing the residual weak acid with an alkaline solid salt.

**5.** A method for preparing an organosilicon resin composition as set forth in claim 1, comprising the steps of subjecting a hydrolyzable silicon compound to hydrolysis and condensation in the presence of an acid catalyst in a solventless system or in an aliphatic alcohol solvent having 1 to 10 carbon atoms, and adding thereto an

organopolysiloxane solvent selected from
a cyclic organopolysiloxane of the following general formula (2):

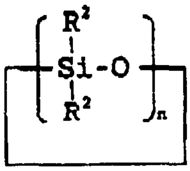

(2)

wherein $R^2$ is independently a substituted or unsubstituted monovalent hydrocarbon group, and n is a number of at least 4, and a linear organopolysiloxane having a viscosity of up to 20 centistokes at 25°C, to effect solvent exchange, thereby yielding a solution in the organopolysiloxane solvent of an organosilicon resin of the following general formula (1):

$$R^1_a(R^1O)_bSiO_{(4-a-b)/2} \quad (1)$$

wherein $R^1$ is independently hydrogen or a substituted or unsubstituted monovalent hydrocarbon group of 1 to 10 carbon atoms, letter a is a positive number of from 0.1 to 3.0 and b is a positive number of from 0.01 to 0.5, and wherein
the hydrolytic condensation is followed by the steps of neutralizing the acid catalyst with an excess amount of an aqueous alkali solution, adding a weak acid to the solution so as to be acidic, and neutralizing the residual weak acid with an alkaline solid salt.

**6.** A cosmetic composition comprising 0.01 to 50% by weight of the organosilicon resin composition of claim 1 or the organosilicon resin of formula (1) obtained by removing the solvent therefrom.

European Patent Office

# EUROPEAN SEARCH REPORT

Application Number
EP 00 30 1224

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | CHEMICAL ABSTRACTS, vol. 124, no. 22, 27 May 1996 (1996-05-27) Columbus, Ohio, US; abstract no. 290606, XP002136910 * abstract * | 1-6 | C08L83/04<br>C08G77/32 |
| D | & JP 08 027272 A (SHIN ETSU CHEM CO LTD) 30 January 1996 (1996-01-30) --- | | |
| A | US 5 070 175 A (TSUMURA HIROSHI ET AL) 3 December 1991 (1991-12-03) * column 1, line 7 - line 12 * * example 1 * --- | 1-5 | |
| A | US 4 268 313 A (PARK DONG-SIL ET AL) 19 May 1981 (1981-05-19) * column 1, line 6 - line 10 * * example 1 * ----- | 1-5 | |

| TECHNICAL FIELDS SEARCHED (Int.Cl.7) |
|---|
| C08G<br>C08L |

The present search report has been drawn up for all claims

| Place of search | Date of completion of the search | Examiner |
|---|---|---|
| BERLIN | 3 May 2000 | Hoepfner, W |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT ON EUROPEAN PATENT APPLICATION NO.** EP 00 30 1224

This annex lists the patent family members relating to the patent documents cited in the above-mentioned European search report.
The members are as contained in the European Patent Office EDP file on
The European Patent Office is in no way liable for these particulars which are merely given for the purpose of information.

03-05-2000

| Patent document cited in search report | Publication date | Patent family member(s) | Publication date |
|---|---|---|---|
| JP 8027272 A | 30-01-1996 | NONE | |
| US 5070175 A | 03-12-1991 | JP 1910836 C | 09-03-1995 |
| | | JP 4033926 A | 05-02-1992 |
| | | JP 6033335 B | 02-05-1994 |
| US 4268313 A | 19-05-1981 | NONE | |

EPO FORM P0459

For more details about this annex : see Official Journal of the European Patent Office, No. 12/82